# EUROPEAN PATENT APPLICATION

(11) **EP 4 357 450 A1**
(43) Date of publication of application: **24.04.2024**
(21) Application number: 23204298.6
(22) Date of filing: 18.10.2023
(51) Int. Cl.: C12N 9/00, C12N 15/82

(54) **PRODUCTION OF RECOMBINANT HUMAN A-THROMBIN IN PLANT-BASED SYSTEM AND THE APPLICATION THEREOF**

(30) Priority: 18.10.2022 US 202263417261 P
(71) Applicant: PROVIEW-MBD BIOTECH CO., LTD., Taipei City (TW)
(72) Inventor: CHANG, YU-CHIA, Taipei City (TW); KUO, JER-CHENG, Taipei City (TW); SU, RUEY-CHIH, New Taipei City (TW); HUANG, LI-KUN, Hsinchu County (TW); LIAO, YA-YUN, Hsinchu City (TW); LEE, Ching-I, Zhubei City (TW); HUNG, SHAO-KANG, Hsinchu City (TW)
(74) Representative: dompatent von Kreisler Selting Werner - Partnerschaft von Patent- und Rechtsanwälten mbB

(57) **Abstract**

The present invention relates to a method for producing recombinant human prethrombin-2 protein and having human α-thrombin activity by the plant-based expression systems.

## Description

### BACKGROUND

### 1. Technical Field

The present invention relates to apply the plant-based expression systems to produce recombinant human prethrombin-2 protein in transient expression of tobacco (*Nicotiana benthamiana*) by *Agrobacterium-mediated* vacuum infiltration. The pure human prethrombin-2 after extraction, purification and factor Xa digestion had human α-thrombin activity.

### 2. Related Art

The blood coagulation cascade is a complex system comprising a series of physiological process which changes blood from liquid to gel and eventually forms the blood clot. The blood coagulation cascade involves in two major pathways: the extrinsic pathway (Tissue factor pathway) and the intrinsic pathway (The contact pathway). The extrinsic pathway is triggered by the tissue factor (TF) when injury and then forms the TF:FVIIa complexes. By contrast, the intrinsic pathway consists of Factor XII (FXII), high molecular weight kininogen, prekallikrein, and Factor XI (FXI). Both pathways interact with several coagulation factors to catalyze a series of downstream reactions which accelerate coagulation.

Thrombin (Coagulation FIIa or fibrinogenase) as an endolytic serine protease is the final enzyme between two pathways of blood coagulation cascade and end up to stopping bleeding (Hemorrhages). It plays a key role in the conversion of fibrinogen into fibrin and binds to platelet plug during the blood coagulation cascade. Additionally, α-thrombin is a cells chemoattractant engaging in phagocytosis, angiogenesis, and binds growth factors to support the wound healing. In blood, α-thrombin is produced from an inactive zymogen prothrombin which is cleaved in two sites by factor V. The multi-domain structure of prothrombin is approximately 72 k*Da* inclusive of an N-terminal Gla domain, two Kringle domain and C-terminal serine protease domain. Cleavage prothrombin at Arg-271 generates the intermediate form, prethrombin-2, containing an A chain (Light chain, ~36-residue) and a B chain (Heavy chain, ~259-residue) connected by a disulfide bond. The prehrombin-2 consequently cleaves at Arg-320 to separate light and heavy chain and result in the α-thrombin. The glycoprotein of α-thrombin comprises an N-linked glycosylation site at Asn-416 and active thrombin linked specific glycan.

In many surgical procedures, hemostasis is essential for patients to decrease the negative impact on wound healing and infection. Hence, numbers of hemostasis agents and devices have been developed to minimize blood loss. The treatment for bleeding disorders is currently restricted to protein replacement therapy, either plasma-derived or recombinant products. So far, three thrombin products consisting of bovine thrombin, human thrombin, and recombinant thrombin have been approved by the FDA for topical use only.

Bovine thrombin is the first and commercialized product for over 60 years, however the product of bovine thrombin has been reported the formation of antibodies might cross react with human coagulation factors. This has been associated with life threatening bleeding and in some circumstances anaphylaxis and death. On the other hand, human thrombin is isolated from pooled human plasma that all donors have been tested for blood-borne disease, e.g. HIV, hepatitis B and C, ... etc. The blood was processed through a series of separation and filtration steps to minimize these risks. However, no any convincing evidence shows that the pathogens and viruses have been completely removed from human plasma. Another problem of the human blood products is insufficient for the potential demand. In 2008, recombinant α-thrombin as the first approval product in CHO cells and is basically free of human-derived contaminants. Nonetheless, the final product of α-thrombin need to be obtained by ecarin (*Echis carinatus*) digestion of prothrombin and the cost is high.

To avoid the risk of viral transmission and minimize the antigenic properties, the plant-made pharmaceutical proteins as an alternative platform have been on the rise for the past two decades. The first recorded plant-made biopharma product is a chimeric human growth hormone which was produced in transgenic tobacco and sunflower callus in 1986. Moreover, the first marketed plant-made pharmaceutical, taliglucerase alfa, was produced in carrot cells by Pfizer/Protalix biotherapeutics to treat Gaucher's disease and approved by Food and Drug Administration (FDA) in 2012. Hence, plants-based system is utilized as an alternative biopharmaceutical protein platform and shows some advantages on market. For example, the cost-efficient, scalability, flexibility, low contamination risk of human pathogens, the ability to perform post-modifications, and robustness of the system compared to microbial or mammalian cell-based expression systems. Recently, plant molecular farming (PMF) is booming on pharmaceutical proteins inclusive of therapeutic proteins, growth factors, enzymes, antibodies, vaccines, and other commercially viable proteins, some of which are in the pre-clinical and clinical pipeline.

Numerous plant species, e.g. tobacco, alfalfa, rice, potato, tomato, sunflower, carrot, lettuce, strawberry, moss, duckweed, maize, and wheat have been applied to produce recombinant protein products. Plant biopharmaceuticals can be produced by *Agrobacterium-*mediated either nuclear (or chloroplast) stable or transient transformation methods. The stable expression system in different expression strategies have been exploited, such as whole plants or in organ cultures (such as hairy roots) and cell suspension cultures. Furthermore, recently the tobacco *N. benthamiana* has been shown the most widely used protein expression platform in "molecular farming community".

Despite the CHO cells produced recombinant α-thrombin has been approved by FDA in 2008; however, the final product need two purification steps and possess the viral transmission risk. Directly expression prothrombin and prethrombin-2 have been reported in tobacco callus system in 1998, but no specific activity. For the purpose of production of functional α-thrombin, plant specific codon optimize, vacuole targeting tag or ER retention tag were performed.

### SUMMARY

The present invention has successfully expressed recombinant human prethrombin-2 in *N. benthamiana* using a viral-based transient expression system. The expression of prethrombin-2 in *N. benthamiana* plants were quantified and prethrombin-2 purification was characterized in comparison to commercial anti-His antibody. The results indicated that plant expression systems can effectively be used as an alternative to mammalian cell system for production of therapeutic protein.

In embodiment 1, the present application provides a method of making recombinant protein or fragment thereof (MDBprethrombin-2) in a plant comprising:
(a) was expression the intermediate form, prethrombin-2, containing an A chain (Light chain, ~36-residue) and a B chain (Heavy chain, ~259-residue).
(b) In present invention, we designed another N-terminal factor Xa cleavage site and then prethrombin-2 also contained the endogenous factor Xa. The digestion by factor Xa could be maturated and activated a light chain and a heavy chain sequence of the human prethrombin-2 at the same time.

In embodiment 2, the prethrombin-2 could express in three *Agrobacterium* strains including of LBA4404, EHA105, and GV3101, respectively.

In embodiment 2, the prethrombin-2 was expressed in 6, 7, and 8 weeks of *N. benthamiana,* respectively.

In embodiment 3, prethrombin-2 could purify in Ni-sepharose 6 fast flow histidine-tagged protein purification resin and immunoblot analysis indicated the purified prethrombin-2 against anti-His antibody.

In embodiment 4, the application further indicated that the active form of α-thrombin was detected after factor Xa digestion.

### BRIEF DESCRIPTION OF THE DRAWINGS

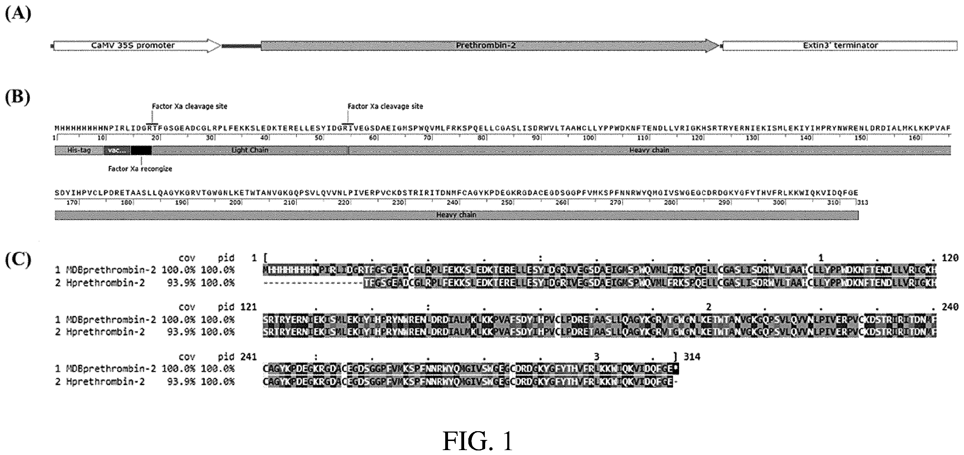

FIG. 1. (A) The gene cassettes of the prethrombin-2 expressed in CaMV 35S and Extin3' terminator (B) The amino acid sequence of prethrombin-2 (MDBprethrombin-2) contains the artificial start codon (methionine, M), recombinant tag for protein purification (8X His tag, HHHHHHHH), vacuole targeting tag for protein trafficking (vacuolar sorting sequence, NPIRL), protease cleavage site for factor Xa (restriction cleavage site, IDGR), and human prethrombin-2. The human prethrombin-2 construct comprises of a light chain and a heavy chain sequence that was maturated and activated by factor Xa digestion. (C) Amino acid sequence alignment of designed construct (MDBprethrombin-2) and wild-type human prethrombin-2 (Hprethrombin-2).

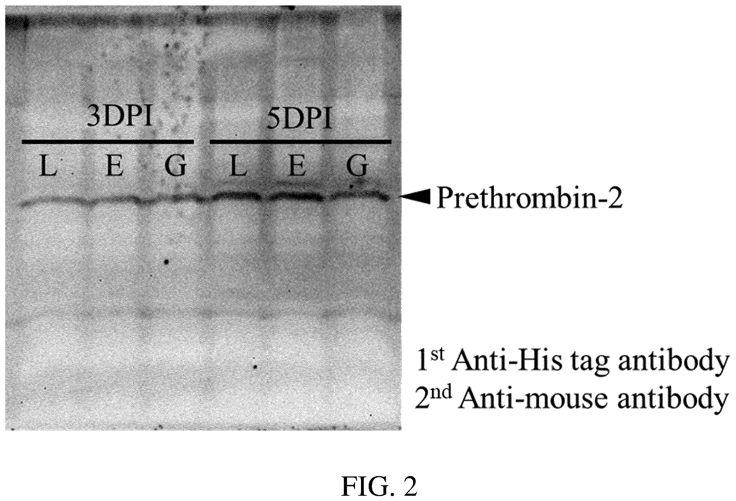

FIG. 2. Immunoblot analysis displayed that the prethrombin-2 expression level of Agro-infected leaves were harvested and extracted after 3DPI and 5DPI (DPI: Day post infiltration). Vacuum infiltration of *N. benthamiana* leaves with three *Agrobacterium tumefaciens* strains containing the prethrombin-2 expressing vector, L: LBA4404, E: EHA105, and G: GV3101, respectively. The arrow shows the Prethrombin-2 against anti-his antibody.

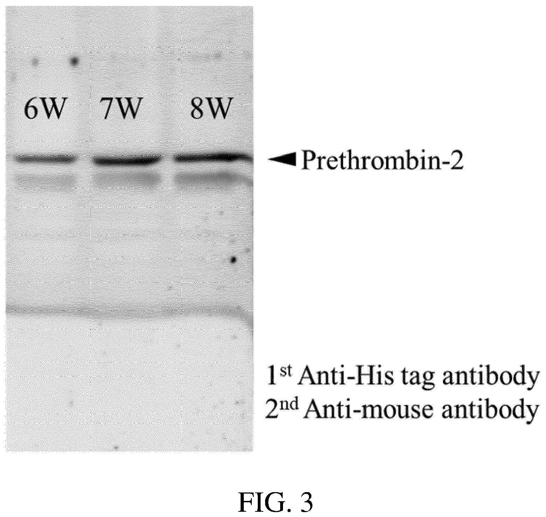

FIG. 3. Immunoblot analysis indicated that the prethrombin-2 was expressed in 6, 7, and 8 weeks of *N. benthamiana,* respectively. The arrow shows the Prethrombin-2 against anti-his antibody.

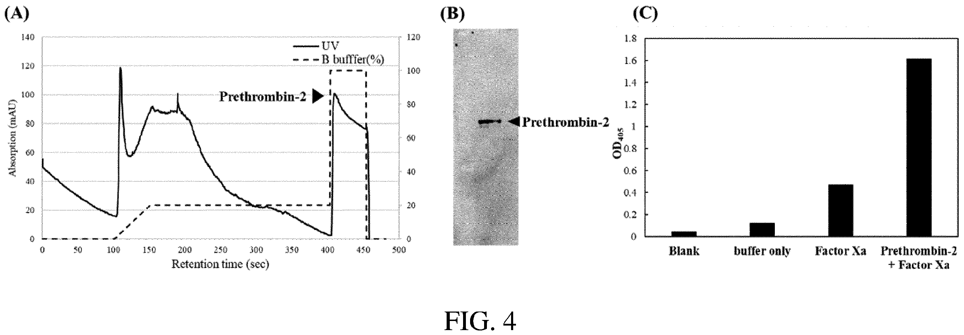

FIG. 4. (A) The 1L sample of tobacco lysate containing His-tag prethrombin-2 was bound to 5 mL Ni-sepharose 6 fast flow histidine-tagged protein purification resin. Bound material was washed by a linear gradient of up to 20% of B Buffer containing 20 mM sodium phosphate, 500 mM NaCl, and 50 mM imidazole, and the final product was eluted by 250 mM imidazole (black triangle). (B) Immunoblot analysis indicated the purified prethrombin-2 against anti-His antibody. (C) Thrombin activity assay measured at OD₄₀₅ nm using S-2238 and the bar chart indicated the relative activity among mock control, factor Xa, and factor Xa+Prethrombin-2.

### DETAILED DESCRIPTION

Human prethrombin-2 was expressed in *N. benthamiana* plants using viral-based transient expression systems. This invention applies to plant expression cassettes encoding CaMV 35S promoter, human prethrombin-2 and Extin3' terminator (FIG. 1A) in tobacco transient system. The designed construct of human prethrombin-2 named MDBprethrombin-2 which fused sequentially with N-terminal the artificial start codon (Methionine, M), 8X His tag for protein purification, vacuole targeting tag (NPIRL) for protein trafficking, factor Xa (IDGR) of protease cleavage site for formation fragment A and B active form (FIG.1B and C). The expression vector containing MDBprethrombin-2 cassette (FIG. 1A) was transformed into three *Agrobacterium tumefaciens* strains LBA4404, EHA105, and GV3101, respectively. LBA4404-prethrombin-2, EHA105-prethrombin-2, and GV3101-prethrombin-2 were confirmed by PCR and cultured for tobacco infection. Vacuum infiltration of *N. benthamian* leaves were harvested and extracted after 3DPI and 5DPI (DPI: Day post infiltration). Immunoblot analysis of crude plant extracts revealed that all of the LBA4404-prethrombin-2, EHA105-prethrombin-2, and GV3101-prethrombin-2 could be expressed in tobacco transient system and the expression level of 5DPI was better than 3DPI (FIG. 2). We also analyzed the growth condition of *N. benthamian* plants among 6, 7, and 8 weeks and immunoblot analysis of crude plant extracts revealed that the expression level of 8 weeks old plant showed better than 6 and 7 weeks old plant (FIG. 3).

Purification of human prethrombin-2 from *N. benthamian* leaves was bound to the affinity chromatography of Ni-sepharose 6 beads fast flow. Bound sample was washed in a linear gradient containing 20 mM sodium phosphate, 500 mM NaCl, and 50 mM imidazole, and the final product was eluted by 500 mM imidazole (FIG. 4A). The purification of prethrombin-2 was achieved FPLC and immunoblot analysis indicated the band of prethrombin-2 against anti-his antibody (FIG. 4B). Thrombin activity assay was measured by S-2238 substrate (H-d-Phe-Pip-Arg-p-nitroanilide, 150 µM; Chromogenix, Molndal, Sweden). Functional assay of individual sample including of mock control, factor Xa control, and factor Xa+prethrombin-2 were reacted at 37°C 30 min and measured at OD₄₀₅ nm (FIG. 4C).

Accordingly, the present invention provides a method of making a recombinant prethrombin-2 and fragment thereof that active α-thrombin form with factor Xa. As we know, full length prothrombin consists of four domains including an N-terminal Gla domain, two kringle sites, and a C-terminal trypsin-like serine protease domain containing 622 a.a. and the M.W of approximately 72kDa. Prothrombinase is composed of factor Xa, Va, and phospholipids with lower catalytic efficacy on prethrombin-2 than prothrombin. Generally, the ecarin specifically converts prethrombin-2 into active α-thrombin, is a snake venom-derived protease, this process is an efficient protease to activate thrombin. However, ecarin is not cost-effective and demands extra time and many stages. Hence, many studies aim to address these limitations to reduce the final cost of thrombin production and made this faster with more production yield. In present invention, we expressed the partial prethrombin-2 (295 a.a.) instead of prothrombin (622 a.a) in tobacco and only used the factor Xa to digest the active form of α-thrombin. FIG.4C shows The relative thrombin activity was measured at OD₄₀₅ containing the mock (1 fold), factor Xa (5.4 fold), and the prethrombin-2+factor Xa (20.1 fold), respectively (FIG. 4C). The data indicates the activity of prethrombin-2+factor Xa could be expressed, purified, and was active in plant system.

### EXPERIMENTAL SECTION

The invention will be further described by means of the following examples which shall not be interpreted as limiting the scope of the appended claims.

### Example 1

### MDBprethrombin-2 construct design

The prethrombin-2 construct containing the CAMV (Cauliflower mosaic virus) 35S promoter, partial sequence prothrombin (prethrombin-2), and Extin3' terminator shown in FIG. IA. The designed prethrombin-2 (MDBprethrombin-2) including of the artificial start codon (methionine, M), recombinant tag for protein purification (8X His tag, HHHHHHHH), vacuole targeting tag for protein trafficking (vacuolar sorting sequence, NPIRL), protease cleavage site for factor Xa (restriction cleavage site, IDGR), and human prethrombin-2 (295 a.a.). The human prethrombin-2 construct comprises of A chain (light chain, ~36-residue) and a B chain (heavy chain, ~259-residue) connecting the endogenous factor Xa digestion. Our construct design indicated the pure MDBprethrombin-2 that was maturated and activated only by factor Xa digestion (FIG. 1C) without the ecarin digestion and one-step purification in Ni-sepharose 6 fast flow histidine-tagged protein purification resin.

### Example 2

### Production of recombinant prethrombin-2 using a viral-based transient expression system in N. benthamiana

The nucleotide of recombinant prethrombin-2 (MDBprethrombin-2) applied to plant specific codon optimization and confirmed by sequencing. Three *agrobacterium* strains, LBA4404, EHA105, and GV3101, was grown individually on YEP liquid medium containing 25 µg/mL Rifampicin at 28°C overnight. MDBprethrombin-2 was transformed to three *agrobacterium* strains by freeze-thaw method and grew on YEP solid medium containing 25µg/mL 50 mg of Kanamycin and 25 µg/mL Rifampicin at 28°C. A single bacterial colony was spread in antibiotics agar plate and checked with colony PCR. The 3 to 5 single bacterial colonies of LBA4404-prethrombin-2, EHA105-prethrombin-2, and GV3101-prethrombin-2 were inoculated into 3 ml of YEP (primary culture) containing the same antibiotics and grown over night on a rotary shaker at 180 rpm at 28°C, respectively. An aliquot of 1 ml bacterial suspension was added to 100 ml YEM medium with the additional antibiotics and grown overnight. Bacteria were pelleted at 5000xg for 5 min and resuspended in 10 mM MgCl₂, 10 mM MES (pH 5.7) with 150 µM acetosyringone(AS) at a density of 0.1 OD at 600 nm and incubated for 2-3 h at room temperature before vacuum infiltration. The 6-, 7- , 8-old-week of *N. benthamiana* in each *Agrobacterium* suspension and were put into a laboratory desiccator connected to a vacuum pump, until the vacuum reached 12 Psi. The 12 Psi vacuum maintained for 1 min followed by a slow release of vacuum and this procedure repeated twice. The infiltration samples were then incubated in a chamber at 25 °C in the light/dark period of 16/8 h. 3DPI and 5DPI samples were harvest and extract the total protein by homogenizing leaves with extraction buffer (50 mM Tris-HCl, 300 mM NaCl, 5 mM EGTA, 0.3mg/ml PMSF). The crude extract was clarified by centrifugation at 14,000xg for 10 min at 4°C.

### Example 3

### Purification of prethrombin-2

The prethrombin-2 was purified with IMAC (Immobilized Metal Chelate Affinity Chromatography) in AKTA UPC10 (FIG.4A). The supernatants of plant were bound through the nickel column and washed by 50 mM imidazole. All of the prethrombin-2 was eluted by 250 mM imidazole, and the final buffer replaced in PBS. Immunoblot analysis showed that the purified prethrombin-2 detected against the Anti-His antibody (FIG.4B).

### Example 4

### Functional assay the prothrombin-2 to active α-thrombin

The α-thrombin was activated by Factor Xa Protease (New England Biolabs), and the specific activity was measured by Chromogenix (Thrombin Substrate S2238). The method for the determination of activity is based on the difference in absorbance (optical density) between the pNA formed and the original substrate. The purified prethrombin-2 was mixed with Factor Xa and 1 mmol/L Chromogenix in PBS. The assays were carried out in 200 ul well in 96 well ELISA plate at 37°C in assay buffer (50 mM Tris•HCl, 0.15 M NaCl, pH 7.4). After 30 mins, the thrombin activities were determined by spectrum photometer in 405nm (FIG.4C).

## Claims

1. A method of producing a polypeptide having human α-thrombin activity in plants, comprising:
(1) a gene expression construct comprising a promoter operably linked in the 5'-3 'direction to a polynucleotide sequence encoding the human prethrombin-2 polypeptide, said gene construct further comprises suitable regulatory elements for plant expression and transformation
(2) introducing the gene construct into plant cells, and cultivating said plant cells under suitable conditions,
(3) expression, extraction, and purification of the said polypeptide from the plant cells,
(4) cleavage of prethrombin-2 polypeptide by protease and obtain active α-thrombin protein.

2. The method of claim 1, wherein the polynucleotide sequence encoding a human prethrombin-2 polypeptide.

3. The method of claim 1, wherein the gene expression construct comprising an expression promoter that can act in plant, and is selected from a group consisting of constitutive promoters, e.g. CaMV 35S, actin, ubiquitin... etc.

4. The method of claim 1, wherein the introducing of the gene construct into plant cells includes infiltrating plant tissues and organs with *Agrobacterium* cells in the presence of a surfactant, co-cultivation of plant callus with *Agrobacterium* cells, and particle bombardment-mediated transformation of plant cells, tissues, and organs.

5. The method of claim 1, wherein the plant cells are selected from the group consisting of tobacco, alfalfa, rice, potato, tomato, sunflower, carrot, lettuce, strawberry, moss, duckweed, maize, wheat, and other genetically modifiable plant species.

6. The method of claim 1, wherein the obtained active α-thrombin is further formulated into pharmaceutical compositions for therapeutic applications, including but not limited to blood clotting disorders or wound healing.

7. The method of claim 2, wherein the polynucleotide sequence was optimized according to the plant codon usage.

8. The method of claim 2, wherein the polynucleotide sequence was fused with an ER retention signal peptide.

9. The method of claim 2, wherein the polynucleotide sequence was fused with a vacuolar retention signal peptide.

10. The method of claim 2, wherein the polynucleotide sequence was fused with a protease cleavage site.

11. The method of claim 10, wherein the protease cleavage site can be recognized by proteases, including but not limited to TEV protease, Enterokinase, SUMO Protease, factor Xa... etc.
